# EUROPÄISCHE PATENTSCHRIFT

(11) **EP 2 734 258 B1**
(45) Veröffentlichungstag und Bekanntmachung des Hinweises auf die Patenterteilung: **25.04.2018**
(21) Anmeldenummer: 12720798.3
(22) Anmeldetag: 02.05.2012
(51) Int. Cl.: A61M 25/00, A61M 25/10

(54) **KATHETER, INSBESONDERE VERWEILKATHETER, ZUR BEHANDLUNG VON FUNKTIONSSTÖRUNGEN UND/ODER ERKRANKUNGEN DER BLASE UND/ODER DER PROSTATA**
CATHETER, PARTICULARLY AN INDWELLING CATHETER, FOR TREATING FUNCTIONAL PROBLEMS AND/OR DISEASES OF THE BLADDER AND/OR PROSTATE
CATHÉTER, NOTAMMENT CATHÉTER À DEMEURE, POUR LE TRAITEMENT DE DYSFONCTIONNEMENTS ET/OU DE MALADIES DE LA VESSIE ET/OU DE LA PROSTATE

(30) Priorität: 21.07.2011 DE 102011108130; 22.08.2011 DE 102011110778
(43) Veröffentlichungstag der Anmeldung: 28.05.2014
(73) Patentinhaber: Otto, Ullrich, 34537 Bad Wildungen (DE)
(72) Erfinder: Otto, Ullrich, 34537 Bad Wildungen (DE)
(74) Vertreter: Strehlke, Ingo Kurt
(86) Internationale Anmeldenummer: PCT/EP2012/001875
(87) Internationale Veröffentlichungsnummer: WO 2013/010600

(56) Entgegenhaltungen:
- WO-A1-95/05866
- WO-A2-96/26748
- WO-A2-03/033045
- US-A- 5 007 897
- US-A1- 2002 165 521

## Beschreibung

Die Erfindung betrifft einen Katheter, insbesondere einen Verweilkatheter, bevorzugt zur Behandlung von Funktionsstörungen und/oder Erkrankungen der Blase und/oder der Prostata, vorzugsweise in Verbindung mit oder nach einer Bestrahlungstherapie eines Prostatakarzinoms und/oder zur medikamentösen und/oder physikalischen Behandlung der Prostata und/oder der Blase, mit einem Katheterschlauch und einem distalen Katheteranschluss, wobei ein vom distalen Katheteranschluss bis zu wenigstens einer Ablauföffnung im Bereich des proximalen Endes des Katheterschlauchs reichender erster Kanal vorgesehen ist und wobei ein vom distalen Katheteranschluss bis zu einem befüllbaren Blasenballon im Bereich des proximalen Endes des Katheterschlauchs reichender zweiter Kanal vorgesehen ist, wobei der Blasenballon im eingeführten Zustand des Katheters zur Anordnung in der Blase vorgesehen ist.

Das Prostatakarzinom stellt den häufigsten Tumor des Mannes dar. Aktuell werden in Deutschland pro Jahr ca. 64000 Neuerkrankungen diagnostiziert. Durch die bessere Diagnostik wird das Prostatakarzinom immer häufiger im lokalisierten Stadium diagnostiziert, so dass die Möglichkeit einer Kuration gegeben ist. In der Vergangenheit bestand die Therapie des lokalisierten Prostatakarzinoms nahezu ausschließlich in der radikalen Prostatovesikulektomie.

In den letzten Jahren sind verschiedene konkurrierende Therapieverfahren entwickelt worden, die zu einer veränderten Patientenpräferenz geführt haben. Dabei setzten sich vor allem diejenigen Therapiemaßnahmen durch, bei denen es mit Hilfe von "Hightech-Maßnahmen" gelingt, Karzinomgewebe präzise und punktgenau zu destruieren.

Aufgrund evidenzbasierter Daten konnten sich besonders radiologische Behandlungsverfahren etablieren. Sie erzielen nachweislich eine befriedigende Kontrolle des Tumors.

Die interdisziplinären Leitlinien zur Früherkennung, Diagnose und Therapie der verschiedenen Stadien des Prostatakarzinoms empfehlen daher auch radiologische Therapieverfahren als primäre Behandlung des lokalisierten Prostatakarzinoms. Aus den vorhandenen Literaturdaten lässt sich erkennen, dass der Anteil der radiotherapierten Patienten derzeit ca. 30 % beträgt. In Amerika ist dieses Therapieverfahren seit Jahren etabliert. Dort werden pro Jahr ca. 80.000 von ca. 240.000 neu erkrankten Patienten mit einem lokalisierten Prostatakarzinom bestrahlt.

Allerdings führt die Bestrahlungstherapie zu postradiogenen Funktionsstörungen der Blase und/oder der Prostata, die die Strahlendosis limitiert und die Lebensqualität des Patienten einschränkt.

Die Hauptfunktionsstörungen betreffen die Blasenfunktion. Diese Störungen sind gekennzeichnet durch eine irritative und obstruktive Symptomatik der Blase und der Prostata. Insbesondere werden die Prostata- und die Blasenschleimhaut in Mitleidenschaft gezogen. Die Bestrahlung führt zu einer Reizung der Blasen- und Prostataschleimhaut und vermehrtem Harndrang mit einer irritativen Symptomatik. Im Ergebnis kommt es zu einer Störung der Blasenspeicherphase, die eine erhebliche Drangsymptomatik verursacht. Zusätzlich kann es durch das Ödem der bestrahlten Prostata zu einer Obstruktion kommen. Irritative und obstruktive Komponenten führen zu einer Symptomatik, die eine Pollakisurie, Nykturie, Dysurie und Restharnbildung bis zum Harnverhalt beinhaltet.

Die vorgenannten Blasenfunktionsstörungen werden zur Zeit zum einen medikamentös, vorwiegend per os, also systemisch, oder durch intravesikale Therapien behandelt, wobei die Konzentration dieser Substanzen am Erfolgsorgan gering und/oder die Verweildauer kurz ist.

Die WO 03/033045 A2 betrifft ein Verfahren, eine Vorrichtung sowie Computersoftware zur Entnahme einer Probe *in vivo* und/oder zur Behandlung eines Patienten, umfassend die Positionierung eines länglichen transurethralen Katheters in die prostatische Harnröhre eines Patienten, wobei der Katheter einen Blasenhalteballon und mindestens eine Eintrittsöffnung für eine Bioprobe aufweist.

Weiterhin betrifft die US 5 007 897 A einen Katheter zur Abgabe von Medikamenten in Hohlorgane des Körpers, wobei auch die Behandlung der Prostata mittels des Harnkatheters, welcher einen zusätzlichen Kanal zur Abgabe von Medikamenten über eine poröse Membran aufweist, möglich ein soll.

Die US 2002/0165521 A1 beschreibt eine medizinische Vorrichtung, welche zur Einführung in eine Körperhülle vorgesehen ist und welche einen oder mehrere aufblasbare Ballone aufweist.

Zudem betrifft die WO 96/26748 A2 einen Dauerurinkatheter mit einem aufblasbaren Halteballon, einem Kanal zur Befüllung des Ballons sowie einem Kanal zum Ablass von Urin, wobei der Katheter zusätzlich an der Harnröhrenmündung einen weiteren Halteballon aufweist.

Die WO 95/05866 A1 beschreibt ein Verfahren zur gleichzeitigen Durchführung einer Angioplastie und medikamentösen Behandlung einer lokalisierte Fläche einer verstopften koronaren oder peripheren Arterie.

Aufgabe der vorliegenden Erfindung ist es, einen Katheter der eingangs genannten Art zur Verfügung zu stellen, der eine bessere Therapierung von Funktionsstörungen und Erkrankungen der Blase und der Prostata erlaubt.

Zur Lösung der erfindungsgemäßen Aufgabenstellung schlägt die vorliegende Erfindung einen Katheter nach Anspruch 1 vor; weitere Ausgestaltungen sind Gegenstand der Unteransprüche.

Zur Lösung der zuvor geschilderten Aufgabe schlägt die vorliegende Erfindung - gemäß dem einzigen Aspekt der vorliegenden Erfindung - einen Katheter in Form eines Verweilkatheters zur medikamentösen und physikalischen Behandlung von Funktionsstörungen bzw. Erkrankungen der Blase und der Prostata in Verbindung mit oder nach einer Bestrahlungstherapie eines Prostatakarzinoms, mit einem Katheterschlauch und einem distalen Katheteranschluss, vor,
wobei ein vom distalen Katheteranschluss bis zu wenigstens einer Ablauföffnung im Bereich des proximalen Endes des Katheterschlauchs reichender erster Kanal zum Ablass von Urin vorgesehen ist,
wobei ein vom distalen Katheteranschluss bis zu einem befüllbaren Blasenballon im Bereich des proximalen Endes des Katheterschlauchs reichender zweiter Kanal zur Medikamentenzufuhr zur Therapie der Blase vorgesehen ist, wobei der Blasenballon im eingeführten Zustand des Katheters zur Anordnung in der Blase vorgesehen ist, wobei der Blasenballon eine permeable Wandung zur Medikamentenabgabe aufweist,
wobei ein vom distalen Katheteranschluss bis zu einem befüllbaren Prostataballon im Bereich des proximalen Endes des Katheterschlauchs reichender dritter Kanal zur Medikamentenabgabe zur Behandlung der Prostata vorgesehen ist, wobei der Prostataballon im eingeführten Zustand zur Anordnung im Bereich der Prostata vorgesehen ist, und wobei der Prostataballon eine permeable Wandung zur Medikamentenabgabe aufweist.

Zur Lösung der vorgenannten Aufgabe ist somit erfindungsgemäß bei einem Katheter der eingangs genannten Art vorgesehen, dass der Blasenballon eine permeable Wandung zur Medikamentenabgabe aufweist. Durch die Ausbildung des Blasenballons als Einrichtung zur Medikamentenabgabe wird letztlich ein Reservoir bzw. ein Depot für ein Medikament zur Verfügung gestellt, wobei das Reservoir im Körper des Patienten am Ort des Geschehens, d. h. an der Therapiestelle, zur Verfügung gestellt wird und das Medikament gezielt über eine vorgegebene Dauer abgegeben werden kann.

Die Erfindung ermöglicht damit eine Behandlung für ein definiertes Zeitsegment durch die kontinuierliche Abgabe eines Medikaments über den permeablen Ballon unmittelbar an der Therapiestelle, wobei über die Durchlässigkeit der Wandung des Blasenballons die Abgabemenge des Medikaments pro Zeiteinheit exakt gesteuert werden kann.

Bei dem erfindungsgemäßen Katheter handelt es sich somit um einen Spezial-Verweilkatheter, mit dem durch physikalische und medikamentöse Maßnahmen nicht nur Funktionsstörungen der Blase und der Prostata, sondern auch Erkrankungen wie Blasenkarzinome, Prostatakarzinome oder gutartige Vergrößerungen der Prostata behandelt werden können. Dabei sollte der erfindungsgemäße Verweilkatheter eine maximale Verweildauer von 48 h in der Blase haben.

Im Zusammenhang mit dem erfindungsgemäßen Katheter ist es zudem vorgesehen, dass ein vom distalen Katheteranschluss bis zu einem befüllbaren Prostataballon im Bereich des proximalen Endes des Katheterschlauchs reichender dritter Kanal vorgesehen ist, wobei der Prostataballon im eingeführten Zustand des Katheters zur Anordnung im Bereich der Prostata vorgesehen ist.

Der vorgenannte Prostataballon dient der Kompression und damit der lokalen physikalischen Behandlung der Prostata, aber auch der medikamentösen Behandlung der Prostata durch Einbringen des Medikamentes in den Prostataballon, das über eine permeable Wand in die Prostata diffundiert, wenn er durch den dritten Kanal befüllt wird. Letztlich wird durch den befüllten Prostataballon die Harnröhre im Bereich der Prostata aufgeweitet und medikamentös beeinflusst.

Erfindungsgemäß ist im Bereich der Auslassöffnung ein Prostataballon vorgesehen, wobei der Prostataballon eine permeable Wandung zur Medikamentenabgabe aufweist. Auf diese Weise erfüllt der Prostataballon dann gleichzeitig mehrere Funktionen. Zum einen bewirkt, wie zuvor beschrieben worden ist, die Befüllung des Prostataballons eine Kompression der Prostata und Aufweitung der Harnröhre im Bereich der Prostata. Neben dieser physikalischen Behandlung ergeben sich durch die Medikamentenabgabe über die durchlässige Wandung die zuvor im Zusammenhang mit dem Blasenballlon beschriebenen Vorteile. Der mit Medikament gefüllte Prostataballon stellt damit ebenfalls ein Depot/Reservoir zur Verfügung, das über eine längere Zeitdauer definiert eine bestimmte Menge an Medikament unmittelbar an der Therapiestelle abgibt.

Erfindungsgemäß ist also eine Kombination von Blasenballon mit durchlässiger Wandung zur Medikamentenabgabe einerseits und im Bereich der Prostata befindliche Auslassöffnung zur Medikamentenzuführung über einen dritten Kanal andererseits vorgesehen, wobei die Auslassöffnung zur Medikamentenzuführung letztlich zur Befüllung des Prostataballons dient, über den die Medikamentenabgabe erfolgt. In diesem Falle ist dann bedarfsweise über den Blasenballon und den Prostataballon eine Medikamentenzufuhr bei gleichzeitiger Kompression der Prostata möglich.

Bei einer bevorzugten Ausführungsform der Erfindung ist vorgesehen, dass die Wandung des Blasenballons und/oder die Wandung des Prostataballons derart ausgelegt bzw. ausgebildet ist, dass die Medikamentenabgabe durch Diffusion über einen Zeitraum von größer einer Stunde, vorzugsweise größer acht Stunden und insbesondere über einen Zeitraum zwischen 18 und 60 Stunden, bevorzugt zwischen 24 und 48 Stunden erfolgt. Dabei ist dann bevorzugt die Durchlässigkeit bzw. Permeabilität des betreffenden Ballons auf die Viskosität des zuzuführenden Medikaments abgestimmt. Je größer die Abgabemenge an Medikament pro Zeiteinheit ist, desto größer ist die Durchlässigkeit der Wandung.

Da die Medikamentenabgabe über die Wandung des Blasenballons und/oder die Wandung des Prostataballons erfolgt, ergibt sich über die Abgabezeit eine Verringerung des Lumens des betreffenden Ballons. In diesem Zusammenhang ist es von Vorteil, dass die Wandung des Blasenballons und/oder die Wandung die Prostataballons derart ausgebildet ist, dass trotz der Medikamentenabgabe im befüllten Zustand ein Außendurchmesser von mehr 20 Charr. verbleibt. Dies bedeutet, dass nach Abgabe einer vorgegebenen Menge an Medikament und Reduzierung des maximalen Lumens auf ein Restlumen keine weitere Medikamentenabgabe mehr erfolgt. Der vorgenannte Außendurchmesser von größer 20 Charr. resultiert letztlich daraus, dass der Katheterschlauch in der Regel einen Durchmesser von 16 Charr. bis maximal 18 Charr. hat. Katheterschläuche mit größeren Durchmessern lassen sich in der Regel nicht mehr über die Harnröhre ohne weiteres einführen. Dadurch, dass sich durch die Medikamentenabgabe der Außendurchmesser auf einen Betrag von maximal 20 Charr. verringert, ist sichergestellt, dass sich der Blasenballon nicht aus der Blase herausziehen lässt und der Prostataballon noch eine hinreichende Kompression der Prostata gewährleistet.

Im Zusammenhang mit der vorliegenden Erfindung bietet es sich im Übrigen an, dass der Blasenballon nach Befüllung im Wesentlichen kugelförmig ist. Durch die Kugelform kann die Anschlagfunktion des Blasenballons im eingeführten Zustand in einfacher Weise sichergestellt werden. Demgegenüber bietet es sich an, dass der Prostataballon nach Befüllung im Wesentlichen zylinderförmig ist. Die zylinderförmige Ausgestaltung stellt sicher, dass es zu einer gleichmäßigen Aufdehnung der Harnröhre im Bereich der Prostata kommt. Dabei sollte der Prostataballon in Abhängigkeit der Größe der Prostata des Patienten eine Länge zwischen 2 cm und 6 cm haben. Dies hat zur Folge, dass grundsätzlich eine Mehrzahl von Kathetern mit unterschiedlichen Längen des Prostataballons zur Verfügung gestellt werden können. Im Übrigen ist festgestellt worden, dass der Prostataballon im befüllten Zustand einen maximalen Außendurchmesser zwischen 20 und 30 Charr., vorzugsweise zwischen 22 und 26 Charr. und insbesondere von etwa 24 Charr. haben sollte. Bei Versuchen ist festgestellt worden, dass bei derartigen Durchmessern eine hinreichende, aber gleichzeitig die Prostata nicht schädigende Kompression sowie eine die Harnröhre nicht schädigende Aufweitung erfolgt.

Bei einer weiteren bevorzugten Ausgestaltung der vorliegenden Erfindung ist am proximalen Ende des Katheterschlauchs eine endseitig geschlossene Spitze vorgesehen, die vorzugsweise hinter der Spitze liegende Schlitze aufweist. Durch die Realisierung der seitlichen Schlitzöffnungen hinter der Spitze ergibt sich eine bessere Urinabfuhr aus der Blase. Im Übrigen ist die Spitze des Katheterschlauchs gebogen, was insgesamt das Handling vereinfacht.

Weiterhin bezieht sich die vorliegende Erfindung auf die Verwendung eines Katheters zur lokalen medikamentösen und/oder physikalischen Behandlung von Funktionsstörungen der Blase und/oder der Prostata, vorzugsweise in Verbindung mit oder nach einer Bestrahlungstherapie eines Prostatakarzinoms, mit einem Katheter der vorgenannten Art.

Im Ergebnis wird durch die vorliegende Erfindung ein transurethraler Katheter zur Verfügung gestellt, der zur lokalen, physikalischen und medikamentösen Therapie des Ödems der Prostata und der abakteriellen Prostatitis, die durch Abbauvorgänge des devitalisierten Gewebes entsteht, sowie der radiogenen Urozystitis besonders geeignet ist und der erstmals eine gezielte lokale Therapie der Blasenfunktionsstörungen nach Radiotherapie des lokalisierten Prostatakarzinoms erlaubt. Über die einzelnen Kanäle dieses 3-Kanal-2-Ballon-Katheters kann den jeweiligen Ballons das Medikament zur Therapie der Urozystitis zugeführt werden. Natürlich können auch andere Medikamente, insbesondere lipophile Substanzen eingesetzt werden, die antiphlogistisch oder antiödematös wirken. Durch die Medikamentenzugabe über die Wandungen des oder der Ballons ist eine über einen langdauernden Zeitraum begrenzte Medikamentengabe möglich. Die Medikamente befinden sich dabei unmittelbar an der Therapiestelle und werden nicht, wie bei der oralen Therapie, über den ganzen Körper verteilt. Durch die Konzentration des Medikaments auf das Zielorgan werden Medikamentenkosten eingespart und systemische unerwünschte Wirkungen vermieden. Die vorliegende Erfindung kann sogar auch zu einer Erhöhung der Behandlungsdosis bei der Radiotherapie führen, da die durch die Radiotherapie verursachten Beeinträchtigungen besser beseitigt werden können. Dies führt letztlich zu einer objektiv besseren Behandlung im Sinne des onkologischen Ergebnisses.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in Ansprüchen oder deren Rückbeziehung.

Es zeigt
- Fig. 1: eine schematische Darstellung eines erfindungsgemäßen Katheters im eingeführten Zustand,
- Fig. 2: eine Schnittdarstellung eines erfindungsgemäßen Katheters und
- Fig. 3: eine schematische Darstellung eines Katheters mit gefülltem Blasenund Prostataballon.

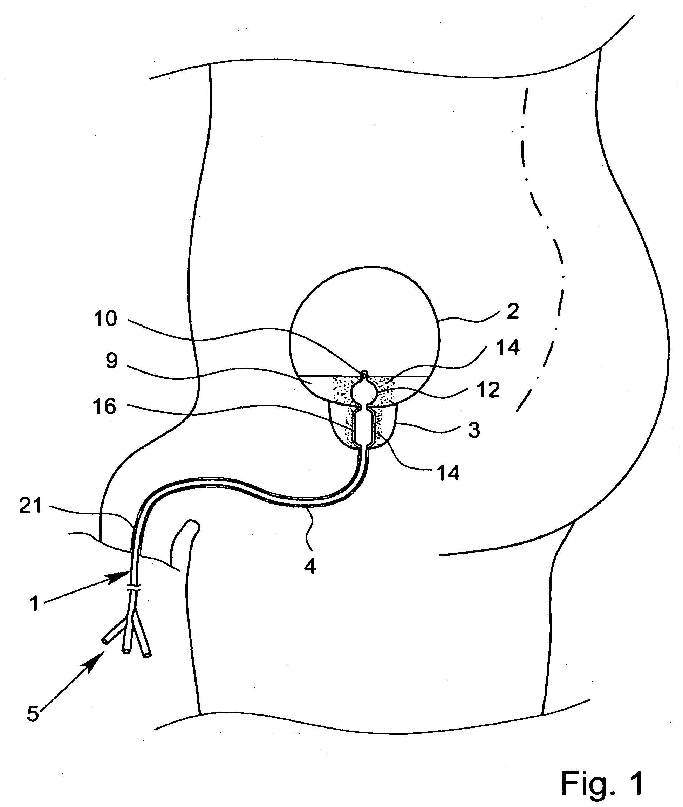

In Fig. 1 ist schematisch ein Katheter 1 im eingeführten Zustand dargestellt. Bei dem Katheter 1 handelt sich um einen Verweil-Blasenkatheter, der insbesondere zur Behandlung von Funktionsstörungen und Erkrankungen der Blase 2 sowie der Prostata 3, insbesondere im Zusammenhang mit einer Bestrahlungstherapie eines Prostatakarzinoms, dient. Der Katheter 1 wird hierzu für ein vorgegebenes Zeitsegment, das maximal 48 h betragen sollte, in die Blase eingeführt.

Der Katheter 1 weist einen Katheterschlauch 4 und einen distalen Kathteranschluss 5 mit vorliegend drei Anschlussenden auf. Der Katheterschlauch 4 weist einen äußeren Mantel 6 auf, der als solcher aus Silikon besteht. Der Außendurchmessers des Katheterschlauchs 4 beträgt ca. 18 Charr. (Charrière). Der mittlere Katheteranschluss ist größer als 18 Charr. und verjüngt sich konisch auf ca. 18 Charr.

Der Katheter 1 selbst hat eine Länge zwischen 20 bis 40 cm.

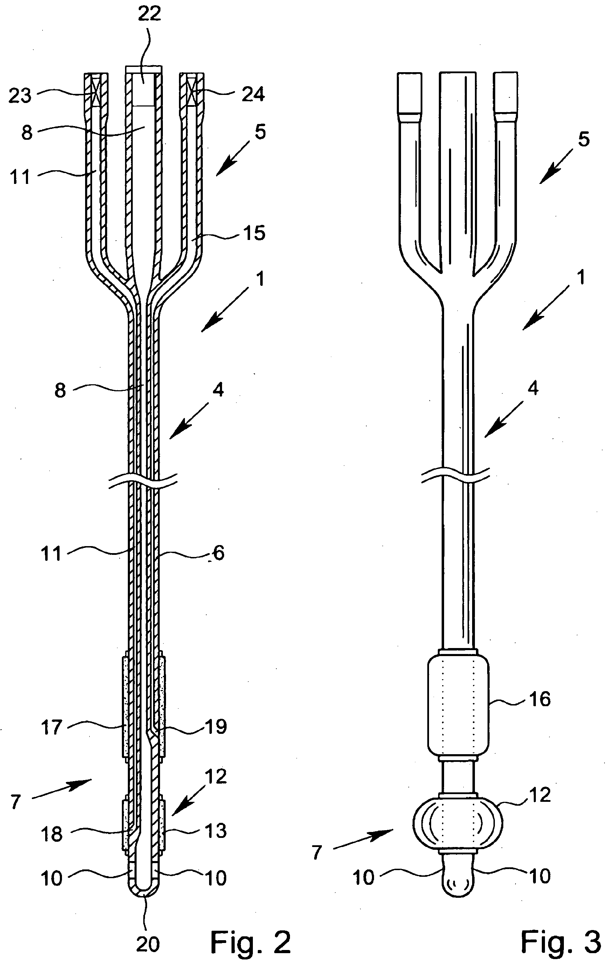

Der Katheter 1 gemäß Fig. 2 weist einen ersten Kanal 8 auf, der vom distalen Katheteranschluss 5 bis zu mehreren Ablauföffnungen 10 im Bereich des proximalen Endes 7 verläuft. Die Ablauföffnung 10 und der erste Kanal 8 dienen zum Ablass von Urin 9 aus der Blase 2.

Weiterhin ist ein zweiter Kanal 11 vorgesehen, der vom distalen Katheteranschluss 5 bis zu einem befüllbaren Blasenballon 12 im Bereich des proximalen Endes 7 des Katheterschlauchs 4 reicht. Der Blasenballon 12 ist vorliegend kugelförmig ausgebildet. Das Lumen des Blasenballons 12 beträgt vorliegend etwa 30 ml. Im eingeführten Zustand des Katheters 1 entsprechend Fig. 1 befindet sich der Blasenballon 12 in der Blase 2 und dient als Anschlag zur Verhinderung des unbeabsichtigten Entfernens/Herausziehens des eingeführten Katheters 1.

Bei der dargestellten Ausführungsform ist es nun so, dass der Blasenballon 12 eine permeable Wandung 13 zur Abgabe eines oder mehrerer Medikamente 14 aufweist. Damit ist die Wandung 13 für das Medikament durchlässig.

Weiterhin weist der Katheter 1 einen dritten Kanal 15 auf, der vom distalen Katheteranschluss 5 bis zu einem befüllbaren Prostataballon 16 im Bereich des proximalen Endes 7 des Katheterschlauchs 4 reicht. Wie sich aus Fig. 1 ergibt, ist der Prostatablallon 16 im eingeführten Zustand zur Anordnung im Bereich der Prostata 3 vorgesehen. Im dargestellten Ausführungsformbeispiel weist der Prostataballon 16 eine permeable Wandung 17 zur Medikamentenabgabe auf. Letztlich ist die Wandung 17 des Prostataballons 16 in gleicher Weise ausgebildet wie die Wandung 13 des Blasenballons 12. Grundsätzlich können die Wandungen 13, 17 auch eine unterschiedliche Durchlässigkeit haben, wenn eine bestimmte Mengenabgabe des Medikaments pro Zeiteinheit über die jeweilige Wandung 13, 17 gewährleistet werden soll. Die Permeabilität der jeweiligen Wandung 13, 17 kann durch die Porosität, insbesondere Porengröße des Materials der Wandung 13, 17, gezielt eingestellt bzw. maßgeschneidert werden, insbesondere in Abhängigkeit von dem zu verabreichenden Medikament 14 bzw. dessen Molekülgröße.

Konkret ist die Ausbildung des Blasenballons 12 so, dass die Wandung 13 auf den Mantel 6 aufgebracht und an den äußeren Rändern des Mantels 6 umlaufend mit dem Mantel 6 verbunden ist. Der zweite Kanal 11 mündet über eine Auslassöffnung 18 in den Ringraum zwischen der Wandung 13 und dem Mantel 6. In ähnlicher Weise ist der Prostataballon 16 realisiert. An den äußeren Rändern ist die Wandung 17 mit dem Mantel 6 umlaufend verbunden. In den Ringraum zwischen der Wandung 17 und dem Mantel 6 mündet eine Auslassöffnung 19 des dritten Kanals 15.

Der lichte Abstand a des Prostataballons 16 zum Blasenballon 12 beträgt vorliegend etwa 1,5 cm. Durch diesen Abstand ist letztlich die exakte Anordnung des Prostataballons 16 im Bereich der Prostata 3 sichergestellt.

Bei den dargestellten Ausführungsformen sind die Wandungen 13, 17 der Ballons 12, 16 derart ausgebildet, dass die Medikamentenabgabe durch Diffusion über einen längeren Zeitraum erfolgt. Die Abgabe des bzw. der Medikamente 14 aus den Ballons 12, 16 erfolgt also *per diffusionem*; die Permeabilität der Ballons 12, 16 bzw. deren Wandungen 13, 17 kann in Abhängigkeit von dem Medikament 14 bzw. dessen Molekülgröße gezielt gewählt werden, insbesondere durch Steuerung der Porengröße des Materials der Wandungen 13, 17 der Ballons 12, 16. Bevorzugt sollte die Medikamentenabgabe über einen Zeitraum zwischen 24 Stunden und 48 Stunden erfolgen. Die Medikamentenabgabe pro Zeiteinheit lässt sich jedenfalls auch durch eine bestimmte Materialwahl der Wandungen 13, 17, deren Porosität oder Permeabilität beeinflussen. So kann es sich bei den Wandungen 13, 17 beispielsweise um permeable Membranen handeln. Die Wandungen 13, 17 sind dabei bevorzugt so ausgestaltet, dass nach der anfänglichen Befüllung der Ballons 12, 16 ein Medikament 14 abgeben wird, so dass das jeweilige Lumen der Ballons 12, 16 sich verringert, dass bei Erreichen eines bestimmten Lumens jedoch keine weitere Medikamentenabgabe mehr erfolgt, wenn der Austausch der Substanzen bzw. Medikamente erfolgt ist und die Konzentration dieser Substanzen, sowohl in dem Ballon oder dem Gewebe der Prostata oder der Flüssigkeit in der Blase zumindest im wesentlichen gleich ist (Ausgleich der Konzentration durch Diffusion). Auf diese Weise verbleibt ein Restlumen der Ballons 12, 16, wodurch sichergestellt ist, dass der Blasenballon 12 weiterhin als Anschlag in der Blase 2 wirken kann und der Prostataballon 16 ebenfalls in Position im Bereich der Prostata 3 gehalten wird und dort die Kompressionswirkung auf das Gewebe der Prostata 3 ausübt.

Wie sich aus den Fig. 1 und 3 ergibt, ist der Blasenballon 12 nach Befüllung im Wesentlichen kugelförmig. Demgegenüber ist der Prostataballon 16 nach Befüllung im Wesentlichen zylinderförmig. Die Länge des Prostataballons 16 kann variieren. Sie hängt letztlich von der Größe der Prostata des jeweiligen Patienten ab. Übliche Längen liegen zwischen 2 und 6 cm. Im befüllten Zustand, wie in den Fig. 1 und 3 dargestellt ist, weist der Prostataballon 16 einen Außendurchmesser von etwa 24 Charr. auf.

Insbesondere die Fig. 2 verdeutlicht, dass am proximalen Ende 7 des Katheterschlauchs 4 eine endseitig geschlossene Spitze 20 vorgesehen ist. Vor der Spitze 20 befinden sich im vorliegenden Fall zwei Ablauföffnungen 10, die rund oder schlitzförmig sein können.

Zur Anwendung des Katheters 1 wird dieser zunächst über die Harnröhre 21 eingeführt, bis sich der Katheterschlauch 4 mit dem noch nicht befüllten Blasenkatheter 12 in der Blase 2 befindet. Sobald sich die Spitze 20 mit den Ablauföffnungen 10 in der Blase 2 befindet, kann etwaig dort befindlicher Urin 9 über die Ablauföffnungen 10 und den ersten Kanal 8 ablaufen. Ein im Bereich des Katheteranschlusses 5 befindlicher Stöpsel 22 dient zum Verschluss des ersten Kanals. Der Stöpsel 22 kann grundsätzlich jederzeit entfernt werden. Grundsätzlich ist auch möglich, einen Stöpsel mit Ventilmechanismus zur bedarfsweisen Entleerung der Blase vorzusehen. Anschließend wird der Blasenballon 12 über den zweiten Kanal 11 mit einem Medikament, beispielsweise ein Medikament 14 zur Therapie der Urozystitis, befüllt. Ein im Bereich des Katheteranschlusses 5 im zweiten Kanal 11 befindliches Ventil 23 verhindert den Rücklauf des betreffenden Medikaments 14. Anschließend wird der Prostataballon 16 über den dritten Kanal 15 mit einem Medikament 14 befüllt. Hierbei handelt es sich um ein Medikament 14 zur Behandlung der Prostata. Im Bereich des Katheteranschlusses 5 befindet sich im dritten Kanal 15 ein weiteres Ventil 24 zur Verhinderung des Auslaufens des Medikaments 14.

Im befüllten Zustand hat der Katheter 1 folgende Funktionen:
1. Durch die Füllung des Prostataballons 16 wird die Prostata 3 komprimiert und das Ödem herausgepresst (physikalischer Ansatz).
2. Es erfolgt eine lokale Therapie der Prostata 3, die durch Abräumvorgänge der devitalisierten Zellen entzündlich verändert ist. Dabei können die lipophilen Substanzen eingesetzt werden, die antiphlogistisch und antiödematös wirken (pharmako-therapeutischer Ansatz der odematös und entzündlich veränderten Prostata).
3. Es wird per difusionem aus der permeablen Wandung 13 des Blasenkatheters 12 eine oder mehrere antiinflammatorische Substanzen zur lokalen Therapie der Urozystitis abgegeben.

Hinzuweisen ist darauf, dass es im Übrigen grundsätzlich möglich ist, über den ersten Kanal 8 Medikament 14 der Blase 2 zuzuführen. Dies ist insbesondere dann zweckmäßig, wenn eine größere Menge an Medikament 14 sehr schnell in die Blase 2 einzubringen ist.

### Bezugszeichenliste:

- 1: Katheter
- 2: Blase
- 3: Prostata
- 4: Katheterschlauch
- 5: Katheteranschluss
- 6: Mantel
- 7: proximales Ende
- 8: erster Kanal
- 9: Urin
- 10: Ablauföffnung
- 11: zweiter Kanal
- 12: Blasenballon
- 13: Wandung
- 14: Medikament
- 15: dritter Kanal
- 16: Prostataballon
- 17: Wandung
- 18: Auslassöffnung
- 19: Auslassöffnung
- 20: Spitze
- 21: Harnröhre
- 22: Stöpsel
- 23: Ventil
- 24: Ventil

- a: lichter Abstand

## Patentansprüche

1. Katheter (1) in Form eines Verweilkatheters zur medikamentösen und physikalischen Behandlung von Funktionsstörungen und/oder Erkrankungen der Blase (2) und der Prostata (3) in Verbindung mit oder nach einer Bestrahlungstherapie eines Prostatakarzinoms, mit einem Katheterschlauch (4) und einem distalen Katheteranschluss (5),
wobei ein vom distalen Katheteranschluss (5) bis zu wenigstens einer Ablauföffnung (10) im Bereich des proximalen Endes (7) des Katheterschlauchs (4) reichender erster Kanal (8) zum Ablass von Urin vorgesehen ist,
wobei ein vom distalen Katheteranschluss (5) bis zu einem befüllbaren Blasenballon (12) im Bereich des proximalen Endes (7) des Katheterschlauchs (4) reichender zweiter Kanal (11) vorgesehen ist, wobei der Blasenballon (12) im eingeführten Zustand des Katheters (1) zur Anordnung in der Blase (2) vorgesehen ist,
wobei ein vom distalen Katheteranschluss (5) bis zu einem befüllbaren Prostataballon (16) im Bereich des proximalen Endes (7) des Katheterschlauchs (4) reichender dritter Kanal (15) zur Medikamentenabgabe zur Behandlung der Prostata vorgesehen ist, wobei der Prostataballon (16) im eingeführten Zustand zur Anordnung im Bereich der Prostata (3) vorgesehen ist, und wobei der Prostataballon (16) eine permeable Wandung (17) zur Medikamentenabgabe aufweist, **gekennzeichnet dadurch, dass** besagter zweiter kanal (11) zur Medikamentenzufuhr zur Therapie der Blase vorgesehen ist, und dass besagter Blasenballon (12) eine permeable Wandung (13) zur Medikamentenabgabe aufweist.

2. Katheter nach Anspruch 1, **dadurch gekennzeichnet, dass** der lichte Abstand (a) des Prostataballons (16) zum Blasenballon (12) wenigstens 0,5 cm, vorzugsweise zwischen 1,0 cm und 3,0 cm und insbesondere etwa 1,5 cm beträgt.

3. Katheter nach Anspruch 1 oder 2, **dadurch gekennzeichnet, dass** die Wandung (13) des Blasenballons (12) und die Wandung (17) des Prostataballons (16) derart ausgebildet sind, dass die Medikamentenabgabe durch Diffusion über einen Zeitraum größer 1 Stunde, vorzugsweise größer 8 Stunden und insbesondere über einen Zeitraum zwischen 18 Stunden und 60 Stunden, bevorzugt zwischen 24 Stunden und 48 Stunden erfolgt.

4. Katheter nach einem der vorhergehenden Ansprüche, **dadurch gekennzeichnet, dass** die Wandung (13) des Blasenballons (12) und/oder die Wandung (17) des Prostataballons (16) derart ausgebildet ist, dass nach Medikamentenabgabe im noch befüllten Zustand ein Außendurchmesser von größer 20 Charr. nicht unterschritten wird.

5. Katheter nach einem der vorhergehenden Ansprüche, **dadurch gekennzeichnet, dass** der Blasenballon (12) nach Befüllung im Wesentlichen kugelförmig ist und dass der Prostataballon (16) nach Befüllung im Wesentlichen zylinderförmig ist und/oder dass der Prostataballon (16) in Abhängigkeit der Größe der Prostata (3) eine Länge zwischen 2 cm und 6 cm und/oder im maximal befüllten Zustand einen Außendurchmesser zwischen 20 Charr. und 30 Charr., vorzugsweise zwischen 22 Charr. und 26 Charr. und insbesondere von etwa 24 Charr. aufweist.

6. Katheter nach einem der vorhergehenden Ansprüche, **dadurch gekennzeichnet, dass** am proximalen Ende (7) des Katheterschlauchs (4) eine endseitig geschlossene Spitze (20) vorgesehen ist.

## Claims

1. A catheter (1) in the form of an indwelling catheter for the medical and physical treatment of dysfunctions and/or diseases of the bladder (2) and the prostate (3) in conjunction with or following a therapeutic treatment of a prostate carcinoma, having a catheter tube (4) and a distal catheter connection (5),
wherein a first channel (8) is provided from the distal catheter connection (5) to at least one drainage opening (10) in the region of the proximal end (7) of the catheter tube (4) in order to drain urine,
wherein a second channel (11) is provided from the distal catheter connection (5) to a fillable bladder balloon (12) in the region of the proximal end (7) of the catheter tube (4) wherein, when the catheter (1) has been inserted, the bladder balloon (12) is provided for positioning in the bladder (2),
wherein a third channel (15) is provided from the distal catheter connection (5) to a fillable prostate balloon (16) in the region of the proximal end (7) of the catheter tube (4) in order to deliver medication for the treatment of the prostate wherein, when it has been inserted, the prostate balloon (16) is provided for positioning in the region of the prostate (3), and wherein the prostate balloon (16) comprises a permeable wall (17) for delivering medication, **characterized in that** said second channel (11) is provided for supplying medication for the therapeutic treatment of the bladder, and **in that** said bladder balloon (12) comprises a permeable wall (13) for delivering medication.

2. The catheter as claimed in claim 1,
**characterized in that** the clearance (a) between the prostate balloon (16) and the bladder balloon (12) is at least 0.5 cm, preferably in the range 1.0 cm to 3.0 cm and in particular approximately 1.5 cm.

3. The catheter as claimed in claim 1 or claim 2,
**characterized in that** the wall (13) of the bladder balloon (12) and the wall (17) of the prostate balloon (16) are constructed in a manner such that the medication is delivered by diffusion over a time period of more than 1 hour, preferably more than 8 hours and in particular over a time period in the range 18 hours to 60 hours, preferably in the range 24 hours to 48 hours.

4. The catheter as claimed in any of the preceding claims,
**characterized in that** the wall (13) of the bladder balloon (12) and/or the wall (17) of the prostate balloon (16) are constructed in a manner such that, after delivery of the medication, an outer diametre of greater than 20 charrière (Charr.) in case of a still filled state is not undercut.

5. The catheter as claimed in any of the preceding claims,
**characterized in that** the bladder balloon (12) after filling is essentially spherical and that the prostate balloon (16) after filling is essentially cylindrical and that the prostate balloon (16), depending on the size of the prostate (3), has a length between 2 cm and 6 cm and/or has, in its maximum filled state, an outer diametre between 20 charrière (Charr.) and 30 charrière (Charr.), preferably between 22 charrière (Charr.) and 26 charrière (Charr.) and particularly of about 24 charrière (Charr.).

6. The catheter as claimed in any of the preceding claims,
**characterized in that**, at the proximal end (7) of the catheter tube (4), a tip (20) closed at its end is provided.

## Revendications

1. Cathéter (1) se présentant sous la forme d'un cathéter à demeure pour le traitement médicamenteux et physique de troubles fonctionnels et/ou de maladies de la vessie (2) et de la prostate (3) en liaison avec ou après une thérapie aux rayons d'un carcinome de la prostate, comportant un tuyau de cathéter (4) et un raccordement distal de cathéter (5),
où il est prévu un premier canal (8) d'évacuation d'urine allant du raccordement distal de cathéter (5) jusqu'à au moins un orifice d'écoulement (10) au niveau de l'extrémité proximale (7) du tuyau de cathéter (4),
où un deuxième canal (11) allant du raccordement distal du cathéter (5) jusqu'à un ballon de vessie (12) remplissable au niveau de l'extrémité proximale (7) du tuyau de cathéter (4), le ballon de vessie (12) étant prévu pour être disposé dans la vessie (2) en position introduite du cathéter (1),
où un troisième canal (15) allant du raccordement distal du cathéter (5) jusqu'à un ballon remplissable (16) au niveau de l'extrémité proximale (7) du tuyau de cathéter (4) afin de délivrer des médicaments de traitement de la prostate, le ballon de prostate (16) étant prévu pour être disposé au niveau de la prostate (3) en position introduite et le ballon de prostate (16) présentant une paroi perméable (17) pour la délivrance de médicaments,
**caractérisé en ce que** ledit deuxième canal (11) est prévu pour l'apport de médicaments destinés à la thérapie de la vessie et que ledit ballon de prostate (12) présente une paroi perméable (13) pour la délivrance de médicaments.

2. Cathéter selon le revendication 1,
**caractérisé en ce que** la distance intérieure (a) entre le ballon de prostate (16) et le ballon de vessie (12) s'élève à au moins 0,5 cm, de préférence 1,0 à 3,0 cm et en particulier à environ 1,5 cm.

3. Cathéter selon le revendication 1 ou 2,
**caractérisé en ce que** la paroi (13) du ballon de vessie (12) et la paroi (17) du ballon de prostate (16) sont réalisées de manière à ce que la délivrance de médicaments se fasse par diffusion sur une période de plus de 1 heure, de préférence de plus de 8 heures et en particulier sur une période de 18 heures à 60 heures, de préférence de 24 heures à 48 heures.

4. Cathéter selon l'une quelconque des revendications précédentes,
**caractérisé en ce que** la paroi (13) du ballon de vessie (12) et/ou la paroi (17) du ballon de prostate (16) est/sont réalisées de manière à ce que, après la délivrance de médicaments, un diamètre extérieur de plus de 20 charrières (Charr.) à l'état encore rempli ne soit pas être sous-passé.

5. Cathéter selon l'une quelconque des revendications précédentes,
**caractérisé en ce que** le ballon de vessie (12) après remplissage est sensiblement sphérique et que le ballon de prostate (16) après remplissage est sensiblement cylindrique et/ou que le ballon de prostate (16) présente, en fonction de la taille de la prostate (3), une longueur entre 2 cm et 6 cm et/ou un diamètre extérieur, à l'état maximalement rempli, entre 20 charrières (Charr.) et 30 charrières (Charr.), de préférence entre 22 charrières (Charr.) et 26 charrières (Charr.) et en particulier d'environ 24 charrières (Charr.).

6. Cathéter selon l'une quelconque des revendications précédentes,
**caractérisé en ce qu'**un embout (20) fermé à son extrémité est prévu à l'extrémité proximale (7) du tuyau de cathéter (4).
